# EUROPEAN PATENT APPLICATION

(11) **EP 4 424 812 A1**
(43) Date of publication of application: **04.09.2024**
(21) Application number: 22886934.3
(22) Date of filing: 24.10.2022
(51) Int. Cl.: C12M 1/26, C12N 5/07, C12Q 1/02

(54) **CELL HOLDING MATERIAL, SENSOR, FRAME MEMBER, AND CELL HOLDING METHOD**

(30) Priority: 25.10.2021 JP 2021174130
(71) Applicant: Integriculture Inc., Tokyo 113-0033 (JP)
(72) Inventor: HATANO, Hiroaki, Tokyo 113-0033 (JP); KAWASHIMA, Ikko, Tokyo 113-0033 (JP)
(74) Representative: Hoffmann Eitle
(86) International application number: PCT/JP2022/039472
(87) International publication number: WO 2023/074607

(57) **Abstract**

A cell holding material capable of holding cells, in which a scaffold to which the cells adhere does not become an obstacle to cell observation, and a cell holding method therefor are provided. A certain embodiment of the present invention is a cell holding material, including a frame member for surrounding all or at least part of a planned holding region capable of holding a cell population obtained by aggregating multiple cells, and the cell population in a form of membrane occupying at least part of the planned holding region and being held by the frame member, in which at least part of a margin of the cell population is brought into contact with the frame member, and further a portion occupying the planned holding region in the cell population is in a state of being hung without physical support.

## Description

### Technical Field

The present invention relates to a cell holding material. The present invention further relates to a sensor including a cell holding material.

### Background Art

Cells respond specifically with high sensitivity along with changes in an external environment. It is expected that by detecting this response reaction by changes of the morphology of the cells, the localization or expression level of the fluorescent protein expressed in the cells, or the like, unprecedented high-performance analysis can be realized. Non Patent Literature 1 discloses a fluorescent protein-type sensor that can detect glucose kinetics in living cells. Further, Non Patent Literature 2 discloses an intracellular protein-type sensor that detects a humoral factor such as an epidermal growth factor existing outside cells. In addition to the above, a detection system with B cells of pathogens and biotoxins, utilizing a cellular response mechanism (Non Patent Literature 3), a sensor for detection of aqueous pollutants in an early stage using algal cells (Non Patent Literature 4), and the like have been developed. These sensors using cells can eliminate instability during long-term measurement, such as signal drift, which is a problem with a conventional semiconductor device and a chemical sensor utilizing an enzyme reaction.

Furthermore, when combined with a cell culture technique, the above-described sensors using cells are expected to have an effect that cannot be achieved by a conventional chemical sensor. For example, Patent Literature 1 discloses a low-cost cell culture technique. By combining with such a cell culture technique, it is considered that a sensor with a lower cost than a conventional chemical sensor can be realized.

In such sensors using cells or the like, a method for stably holding the cells at a desired position is required. Non Patent Literature 5 discloses a cell sensor produced by a 3D printer, in which living cells suspended in a gel are placed in multiple chambers each having an inner diameter of about 1 mm.

### Citation List

### Patent Literature

Patent Literature 1: JP 6111510 B1

### Non Patent Literature

Non Patent Literature 1: Mita, et al., "Green Fluorescent Protein-Based Glucose Indicators Report Glucose Dynamics in Living cells", Anal. Chem., 2019, vol. 91, No. 7, p. 4821-4830
Non Patent Literature 2: Kim, et al., "Intensiomertric biosensors visualize the activity of multiple small GTPases in vitro", Nat. Commun., 10, Article number: 211 (2019)
Non Patent Literature 3: Banerjee, ey al., "A novel and simple cell-based detection system with a collagen-encapsulated B-lymphocyte cell line as a biosensor for rapid detection of pathogens and toxins", Lab. Invest., 2008, Feb, 88(2), 196-206
Non Patent Literature 4: Ferro, et al., "Development of a biosensor for environmental monitoring based on microalgae immobilized in silica hygrogels", Sensors (Basel)., 2012, Dec 6, 12(12), 16879-16891
Non Patent Literature 5: Oda, et al., "Cell-based Biohybrid Sensor Device for Chemical Source Direction Estimation", Cyborg and Bionic Systems, Volume 2021, Article ID 8907148

### Summary of Invention

### Technical Problem

In a conventional cell holding technique, since a scaffold to which cells adhere is required, there is a problem that the scaffold to which the cells adhere becomes an obstacle during cell observation. The present invention has been made in view of such circumstances, and an object of the present invention is to provide a cell holding material capable of holding cells, in which a scaffold to which the cells adhere does not become an obstacle to cell observation, and a cell holding method therefor.

### Solution to Problem

As a result of intensive studies, the present inventors have found that the above problem can be solved by immersing a frame member for surrounding all or at least part of a planned holding region of a cell population obtained by aggregating multiple cells that can form a spheroid, in a medium containing the cell population or a spheroid formed of the cell population. Further, the present inventors have also found that according to the above method, cells can be held in the cell holding material over a long period of time, and thus have completed the present invention.

That is, according to a first aspect of the present invention,
a cell holding material, including
a frame member for surrounding all or at least part of a planned holding region capable of holding a cell population obtained by aggregating multiple cells, and
the cell population in a form of membrane occupying at least part of the planned holding region and being held by the frame member,
in which at least part of a margin of the cell population is brought into contact with the frame member, and further a portion occupying the planned holding region in the cell population is hung without physical support is provided.

According to a second aspect of the present invention,
a frame member for holding part of a cell population obtained by aggregating multiple cells while hanging the part without physical support,
in which the frame member surrounds all or at least part of a planned holding region capable of holding the cell population is provided.

According to a third aspect of the present invention,
a cell holding method for holding part of a cell population obtained by aggregating multiple cells while hanging the part without physical support, including
immersing a frame member for surrounding all or at least part of a planned holding region of the cell population in a medium containing the cell population or a spheroid formed of the cell population is provided.

In the above first aspect, an outer periphery of the frame member in a cross section perpendicular to a surface of the planned holding region may be at least partially covered with the cell population.

In the above first aspect, the cell population may occupy 50% or more of an area of the planned holding region.

In the above first aspect, the planned holding region may be a substantially circular shape or a substantially elliptical shape.

In the above first aspect, the frame member may surround all of the planned holding region.

In the above first aspect, cells of the cell population may be cultured primary cells.

In the above first aspect, cells of the cell population may be cells derived from an organ selected from a liver, a stomach, a lung, a heart, or a brain.

In the above first aspect, an area of the planned holding region may be 1000 to 600000 µm².

In the above first, second, and third aspects, an area of the planned holding region may be 1000 to 6000 µm².

In the above first and third aspects, a spheroid formed of the cell population may have a spheroid size of 0.2 to 1.0 mm².

In the above first aspect, the cell holding material may be included in a sensor.

### Advantageous Effects of Invention

According to the present invention, a cell holding material capable of holding cells, in which a scaffold to which the cells adhere does not become an obstacle to cell observation, and a cell holding method therefor can be provided. Further, according to the present invention, a cell holding material capable of holding cells, in which a scaffold to which the cells adhere over a long period of time does not become an obstacle to cell observation, and a cell holding method therefor can be provided.

### Brief Description of Drawings

Fig. 1(a) is a plan view showing an outline of a cell holding material according to First Embodiment, and Fig. 1(b) is a sectional view of the cell holding material shown in Fig. 1(a) along the line A-A'.
Fig. 2 is a plan view showing an outline of a cell holding material according to Second Embodiment.
Fig. 3 is a diagram showing the number of seeded cells and a spheroid size in HEK293T cells in a logarithmic graph.
Figs. 4(a) to 4(f) show optical microscope images of HEK293T cells after 1 day of culture when the area of a planned holding region of a frame member is 1256 µm², 5024 µm², 31400 µm², 125600 µm², 282600 µm², and 502400 µm², respectively.
Figs. 5(a) and 5(b) show a bright field image and a fluorescence image of HEK293T cells after 28 days from the start of culture when the area of a planned holding region of a frame member is 5024 µm².
Fig. 6 is a diagram showing an occupancy rate of each cell population of HEK293T cells after the lapse of each culture period in an area of a planned holding region when the cells are in each area of a planned holding region and have each spheroid size.
Fig. 7 is a diagram showing the number of seeded cells and a spheroid size in liver-derived cells of a chicken fetus (E12) in a logarithmic graph.
Fig. 8 shows optical microscope images of liver-derived cells of a chicken fetus (E12) after the lapse of each culture period.
Fig. 9 shows optical microscope images of cells derived from each organ of a chicken fetus (E12) after 1 day of culture in each area of a planned holding region.
Fig. 10 shows optical microscope images of cells derived from each organ of a chicken fetus (E12) after the lapse of each culture period when the area of a planned holding region is 1256 µm².
Fig. 11 shows optical microscope images of cells derived from each organ of a chicken fetus (E12) after the lapse of each culture period when the area of a planned holding region is 5024 µm².
Fig. 12 shows optical microscope images of cells derived from each organ of a chicken fetus (E12) after the lapse of each culture period when the area of a planned holding region is 31400 µm².
Fig. 13 shows optical microscope images of cells derived from each organ of a chicken fetus (E12) after the lapse of each culture period when the area of a planned holding region is 125600 µm².
Fig. 14 shows optical microscope images of cells derived from each organ of a chicken fetus (E12) after the lapse of each culture period when the area of a planned holding region is 282600 µm².
Fig. 15 shows optical microscope images of cells derived from each organ of a chicken fetus (E12) after the lapse of each culture period when the area of a planned holding region is 502400 µm².
Fig. 16 is a diagram showing an occupancy rate of each cell population in an area of a planned holding region of cells derived from each organ of a chicken fetus (E12) after the lapse of each culture period in each area of the planned holding region.
Fig. 17 shows a bright field image, a fluorescence image, and a merged image thereof when a spongy sponge is used as the frame member of HEK293T cells.

### Description of Embodiments

Hereinafter, the present invention will be described in detail while making reference to drawings. In the drawings referred to below, an element having substantially the same function is indicated by a common reference numeral, and the description may be omitted. Note that the dimension ratios of the drawings cited in the following embodiments are exaggerated for the convenience of explanation, and may differ from the actual ratios. In addition, the drawings cited in the following embodiments are shown by simplifying or illustrating the constitution, or omitting some elements for the convenience of explanation.

### (First Embodiment)

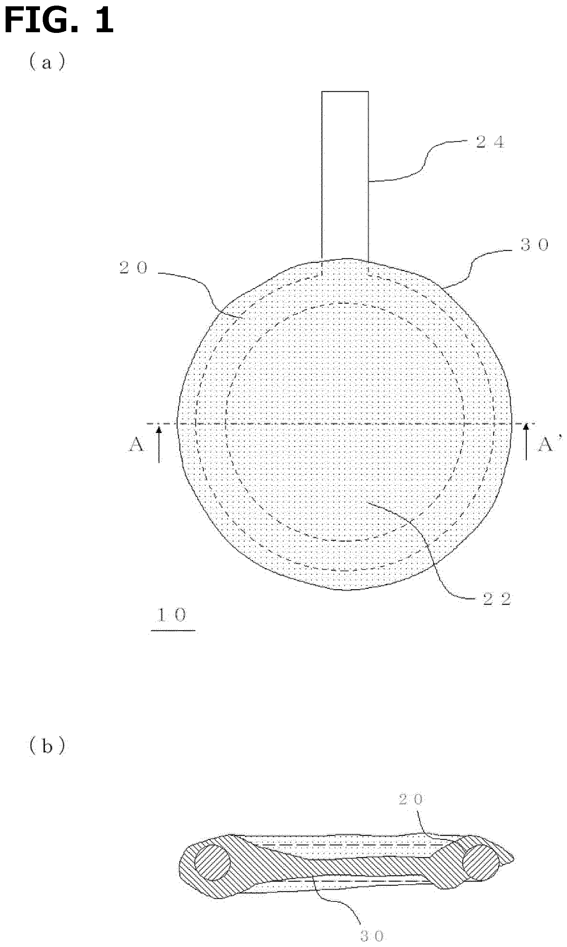

Fig. 1 shows a cell holding material according to First Embodiment. Fig. 1(a) shows a plan view showing an outline of the cell holding material. Fig. 1(b) shows a sectional view of the cell holding material shown in Fig. 1(a) along the line A-A'. As shown in Fig. 1(a), the cell holding material 10 includes a frame member 20, and a cell population 30.

The frame member 20 surrounds all of the planned holding region 22 capable of holding a cell population that is obtained by aggregating multiple cells. Cells and a cell population, which are held by the frame member 20, will be described later.

The material of the frame member 20 is not particularly limited unless it has cytotoxicity. In the present embodiment, the cell population 30 is held by the frame member 20 by at least partially covering the frame member 20. Therefore, the material of the frame member 20 may be a base material having cell adhesiveness, or a base material not having cell adhesiveness. From the viewpoint of enhancing the holding power, a base material having cell adhesiveness is preferable. Examples of the base material that can be used include a polymer material or synthetic resin material such as polyethylene, polypropylene, polyester, polyamide, polyimide, polyurethane, polycarbonate, or polypeptide, an inorganic compound such as stainless steel, apatite, or calcium phosphate, and a natural material such as sponge, cellulose, soy protein, polysaccharides, gelatin, egg white, fungi, or decellularized tissues. From the viewpoint of the cell adhesiveness, the base material is preferably polyimide, or sponge.

The cross-sectional shape of the frame member 20 is a circular shape in Fig. 1(b), but is not particularly limited. Examples of the cross-sectional shape of the frame member 20 include a polygonal shape, a substantially circular shape, and a substantially elliptical shape. The cross-sectional diameter (the maximum width of a cross-sectional length of the frame member perpendicular to a surface of the planned holding region) of the frame member 20 is not particularly limited. The lower limit value of the cross-sectional diameter of the frame member 20 is, for example, 0.1 µm or more, 1 µm or more, 3 µm or more, or 10 µm or more. The upper limit value of the cross-sectional diameter of the frame member 20 is, for example, 10 mm or less, 1 mm or less, or 100 µm or less. The cross-sectional diameter of the frame member 20 in one embodiment can be, for example, 25 µm, or 60 µm.

The planned holding region 22 is an area formed in an inner portion of the frame member 20 in a medium, and may be a flat area or a curved area. Further, in Fig. 1(a), the shape of the planned holding region 22 is a circular shape when the frame member 20 is viewed in plan view, but is not particularly limited. When the frame member 20 is viewed in plan view, examples of the shape of the planned holding region 22 include a polygonal shape, a substantially circular shape, and a substantially elliptical shape, and the shape is suitably a substantially circular shape or a substantially elliptical shape.

The area of the planned holding region 22 is not particularly limited as long as it has a size enough to immerse the frame member 20 in a medium, but is specifically, for example, 1000000 µm² or less, suitably 500 to 600000 µm², more suitably 1000 to 600000 µm², and particularly suitably 1000 to 6000 µm². With such a range of the planned holding region 22, a cell population can be held at a high occupancy rate in the planned holding region.

A bar-shaped bar member 24 is attached to the frame member 20. A base material of the bar member 24 is not particularly limited. The bar member 24 may be formed of the same base material as the frame member 20, and be integrally molded with the frame member 20. The shape of the bar member 24 is not particularly limited as long as it can be gripped. By gripping the bar member 24, the frame member 20 can be easily handled. In other words, by gripping the bar member 24, a cell population 30 held by the frame member 20 can be easily moved. As a result, the cell population 30 is easily subjected to microscopic observation, assay, or the like.

The cell population 30 is a collection of cells in a form of membrane, which occupies at least part of the planned holding region 22 and is held by the frame member 20. In Fig. 1(a), the cell population 30 occupies the entire planned holding region 22, but the cell population 30 may occupy part of the planned holding region 22. The cell population 30 occupies preferably 50% or more, more preferably 80% or more, and furthermore preferably 90% or more of the area of the planned holding region 22, and occupies most preferably all of the planned holding region 22.

At least part of a margin of the cell population 30 is brought into contact with the frame member 20. Chemical bonding between the margin of the cell population 30 and the frame member 20 is not necessarily required. As shown in Fig. 1(b), in a predetermined cross section, the entire outer periphery of the frame member 20 may be covered with part of the cell population 30 (see, the left side of Fig. 1(b)), or part of the outer periphery of the frame member 20 may be covered with part of the cell population 30 (see, the right side of Fig. 1(b)). From the viewpoint of enhancing the holding power, in a predetermined cross section, the entire outer periphery of the frame member 20 is preferably covered with part of the cell population 30.

As shown in Fig. 1(b), in the cell population 30, a portion occupying the planned holding region 22 is hung without physical support. In this regard, the expression "hung without physical support" means that in the cell population 30, one and the other surfaces of the portion occupying the planned holding region 22 are exposed, and is a state in which a space free of interfering objects exists on one surface side and the other surface side of the cell population 30. Consequently, for example, there is no obstacle such as a scaffold when observing cells.

The cells constituting the cell population 30 are not particularly limited as long as they are multiple cells that can form a spheroid. The term "spheroid" means a collection of several or more cells in a substantially spherical form, and is also referred to as an aggregate, or an agglomerated mass. The lower limit value of the number of cells per spheroid that can be formed is, for example, 10 or more, 100 or more, 1000 or more, or 3000 or more. The upper limit value of the number of cells per spheroid that can be formed is, for example, 1000000 or less, 500000 or less, 100000 or less, or 20000 or less. The size of a spheroid is expressed by a spheroid size. The spheroid size means an area (horizontal projection area) inside the outer edge of a spheroid photographed by using a microscope or the like. For example, a phase-contrast microscope can be used to photograph a spheroid, and analysis software can be used to measure the area. As the phase-contrast microscope, for example, an all-in-one fluorescence microscope BZ-X810 (KEYENCE CORPORATION) can be used, and as the analysis software, for example, Leafareacounter Plus can be used. The spheroid size is specifically, for example, 0.01 to 10.0 mm², suitably 0.1 to 3.0 mm², and more suitably 0.2 to 1.0 mm². With such a range of the spheroid size, a cell holding material can hold cells over a long period of time. In this regard, when the cell strain is different, the size per cell is different, so that the number of cells per spheroid may differ even if the size of spheroid is the same. Therefore, it is necessary to set the optimal number of cells for each cell strain.

Further, the cell strain of the cells according to the present embodiment is not particularly limited, and examples of the cell strain include a germ cell such as a sperm or an ovum, a somatic cell constituting a living body, a normal cell line, a cancer cell line, a cultured primary cell, a progenitor cell, a stem cell, a cell isolated from a living body and artificially induced to differentiate, and a cell isolated from a living body and artificially genetically modified. As the cell strain of cells, for example, HEK293T cells can be used. The origin of these cells is not particularly limited, but the cells are preferably cells derived from a mammal such as a human, a rat, a mouse, a guinea pig, or a rabbit, more preferably cells derived from a poultry animal such as a chicken, or a duck, and furthermore preferably cells derived from a chicken. Furthermore, an organ from which cells are derived is not particularly limited, and the cells include liver-derived cells, stomach-derived cells, lung-derived cells, heart-derived cells, brain-derived cells, or the like. In this regard, the cell strain of the cells according to the present embodiment is not necessarily required to be a single kind of cells as long as the cells can form a spheroid, and the cell population 30 may be formed of multiple kinds of cells. Moreover, the occupancy rate of the cell population 30 in the planned holding region 22 differs depending on the binding specificity of cells, and cells with uniform binding specificity are preferable because the occupancy rate increases. As the cells with uniform binding specificity, for example, cultured primary cells being in a group of cells constituting the same organ are mentioned, and in particular, liver-derived, stomach-derived, lung-derived, heart-derived, or brain-derived cultured primary cells are preferable.

### (Cell culture)

Culture of cells can be performed by a known method, for example, under the conditions of 37°C, and 5% CO₂ by using a medium and a culture container that are suitable for the cells to be cultured. Further, the culture method described below includes all of the culture methods of culturing cells in a preparation stage before preparation of a cell holding material, and culturing cells during preparation of a cell holding material. The culture may be any of static culture, shaking culture, spinner culture, or the like. For the culture, an incubator adjusted to a desired temperature, a constant temperature bath, or the like can be used. The culture may be adhesion culture, but it is preferable to culture in a state of non-adhesion for at least part of the period (for example, suspension culture). The term "state of non-adhesion" means a state in which all or most of cells have not adhered to a surface of a culture container, and includes a state in which all or most of cells exist away from a surface of a culture container, and a state in which all or most of cells can be easily separated from a surface of a culture container due to coating of the culture container, convection flow of a medium, or the like, without using any instrument, enzyme, or the like even if the cells are in contact with the surface of the culture container.

The medium may be any medium capable of performing culture of cells, and can be appropriately selected and used according to the cell strain to be used. Examples of the medium include Eagle's minimum essential medium (EMEM), Dulbecco's modified eagle medium (DMEM), RPMI-1640 medium, and Ham's F-12 medium, but are not limited thereto. Further, in these media, known materials and additives useful for cell culture can be appropriately used. Specifically, for example, into these media, a serum, various kinds of growth factors, and a differentiation-inducing factor may be added. As the serum, for example, fetal bovine serum (FBS) can be used. The medium may be changed periodically during the culture period.

As the culture container, any culture container can be used, but in order to promote the formation of a spheroid and/or a cell population, it is preferable that the culture container has not been treated to improve cell adhesiveness, and it is more preferable that the culture container has been subjected to a non (low)-cell adhesion treatment or has been made of a non (low)-cell adhesive material. As the treatment to improve the cell adhesiveness, for example, a coating treatment with an extracellular matrix or the like on a surface of a container can be mentioned. In addition, as the non (low)-cell adhesion treatment, for example, a non-cell adhesive hydrogel coating treatment on a surface of a container, an MPC (2-methacryloyloxyethyl phosphorylcholine) polymer coating treatment, or the like can be mentioned.

### (Cell holding method)

The preparation method of a cell holding material will be described.

First, a medium, cells, and a frame member 20 (see Fig. 1) are prepared. In this regard, the cells to be prepared may be in a state in which individual cells are dispersed before formation of a spheroid, or in a state after formation of a spheroid. Even if the cells are any cells in any state, a cell population 30 is held in the frame member 20.

Next, the frame member 20 is immersed in a medium containing cells (or spheroids). The immersion time of the frame member 20 is not particularly limited, but can be set to, for example, 1 hour or more, 2 hours or more, 5 hours or more, 10 hours or more, or 24 hours or more. In this regard, if a bar member 24 is attached to the frame member 20, it becomes easy to immerse in the present step and to take out in the step described later.

When the frame member 20 is immersed, a cell holding material 10 with a cell population 30 in a form of membrane formed in a planned holding region is obtained. According to the cell holding method described above, a cell holding material can be easily prepared.

With the cell holding material prepared by the above method, a scaffold to which the cells adhere does not become an obstacle to cell observation, and as a result, it becomes easier to manipulate a cell population in micro units. The cell holding material may be used in a prepared culture container, or may be taken out and used. In this regard, it is not essential that the individual cells constituting a cell population are alive at the time of use, and some or all of the cells constituting a cell population can be dead cells.

The cell population held in the cell holding material according to the present embodiment is held over a long period of time. The period to be held is, for example, 14 days or more after the start of holding in one embodiment, and may be 21 days or more or 28 days or more after the start of holding in other embodiments.

### (Second Embodiment)

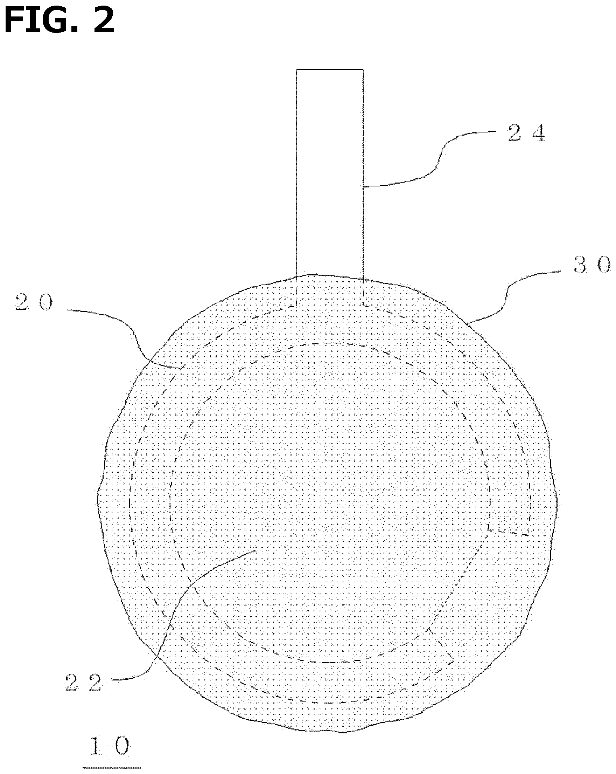

Fig. 2 is a plan view showing an outline of a cell holding material according to Second Embodiment. The basic constitution of the cell holding material 10 according to the present embodiment is similar to that of First Embodiment. Hereinafter, the constitution different from that of First Embodiment will be described.

A frame member 20 according to the present embodiment surrounds part of a planned holding region 22. As described above, in a case where the frame member 20 has an open structure, an area surrounded by the frame member and a line connecting both end points at the shortest distance of the open structure part of the frame member, including the line is regarded as an inner portion of the frame member, and is defined to be a planned holding region 22.

According to the cell holding material 10 of the present embodiment, in a break part of the frame member 20, it is possible to measure the membrane thickness of a cell population 30, and to perform the observation from a direction parallel to the planned holding region 22.

### (Use of cell holding material)

Since the cell holding material according to the present embodiment is simple to manipulate a cell population in micro units, the cell holding material can be used as a cell sensor or the like utilizing physiological activity of cells. In other words, a sensor such as a cell sensor includes the cell holding material according to the present embodiment. As the cell sensor, for example, the presence or absence and/or the content of a medium component can be estimated. Further, the cell holding material can also be used as an intermediate device that converts information from the extracellular environment into electrical signals, optical signals, cell-released substances including extracellular vesicles, or the like. On the other hand, a growth factor and the like can be supplied into a medium from the cells held in the cell holding material. Further, the cell holding material according to the present embodiment can be used as a cell sensor that utilizes activity of a membrane protein such as a transporter internally existing in a lipid bilayer membrane of a cell. In addition, the cell holding material can be used as a molecular recognition element for viruses or bacteria by utilizing a lipid raft formed on a cell membrane of a dead cell held.

The cell holding material according to the present embodiment can be prepared into a multicellular tissue by bringing cell holding materials holding different cell strains into contact with each other. Such a multicellular tissue can be used as a tool for regenerative medicine or pharmacokinetic evaluation.

The present invention is not limited to the embodiments described above, changes such as various design changes can be made based on the knowledge of those skilled in the art, and embodiments to which such changes have been made are also included in the scope of the present invention.

The cell holding material according to the present embodiment may include multiple frame members described above. In a case where multiple frame members are included, the frame members may be in a two-dimensional aligned state of frame material, or may have a three-dimensional network structure.

### Examples

### «Holding of HEK293T cells by frame member made of polyimide»

### <Evaluation of relationship between the number of cells and spheroid size>

In order to evaluate the relationship between the number of seeded cells and a spheroid size, formation of a spheroid of HEK293T cells in which fluorescent proteins were expressed (No. JCRB9068, Independent Administrative Agency, the National Institutes of Biomedical Innovation, Health and Nutrition, JCRB Cell Bank) was induced by using a culture container to which a non (low)-cell adhesion treatment was performed. Specifically, a predetermined number of cells were suspended and seeded in DMEM (Product number: 043-30085, FUJIFILM Wako Pure Chemical Corporation) containing 10% FBS (Product number: S1580-500, Biowest), and the culture was performed under the conditions of 37°C, and 5% CO₂.

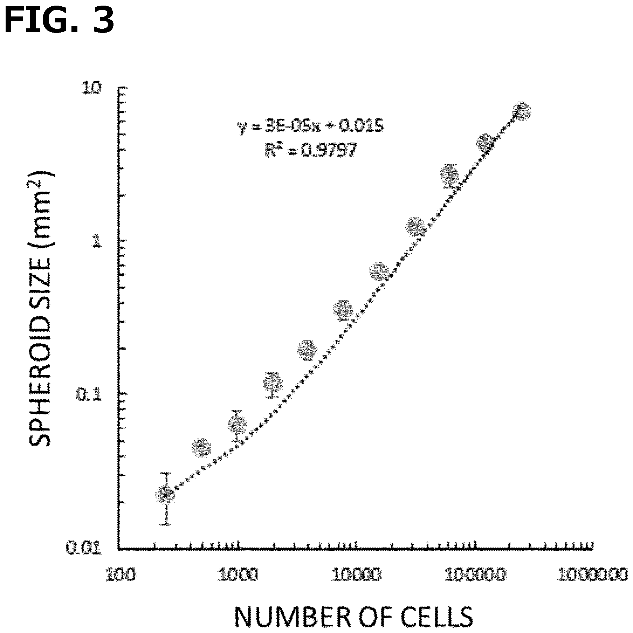

After 1 day from the start of culture, it was confirmed that a spheroid was formed in all of any number of cells. The spheroid size at this time was measured by using analysis software (Leafareacounter Plus) from a photograph taken with an optical microscope (all-in-one fluorescence microscope BZ-X810, KEYENCE CORPORATION). Results at this time of the number of seeded cells and the spheroid size in a logarithmic graph are shown in Fig. 3.

From the results shown in Fig. 3, when the number of seeded cells and the spheroid size were plotted on a logarithmic graph, it was found that there was a linear relationship between them.

### <Evaluation of cell holding>

Fluorescent protein-expressing HEK293T cells were suspended in DMEM containing 10% FBS, and seeded in a culture container to which a non (low)-cell adhesion treatment was performed so that a predetermined spheroid size is obtained. At the same time, as the frame member that surrounds the entire planned holding region having an elliptical shape, LithoLoops Round (Waken Btech Co., Ltd, made of polyimide resin, cross-sectional diameter of frame structure body: 25 µm) with a predetermined area size of the planned holding region was immersed in each well in which cells were seeded (each condition: n = 3). This plate was left to stand in an incubator, the culture was started under the conditions of 37°C, and 5% CO₂.

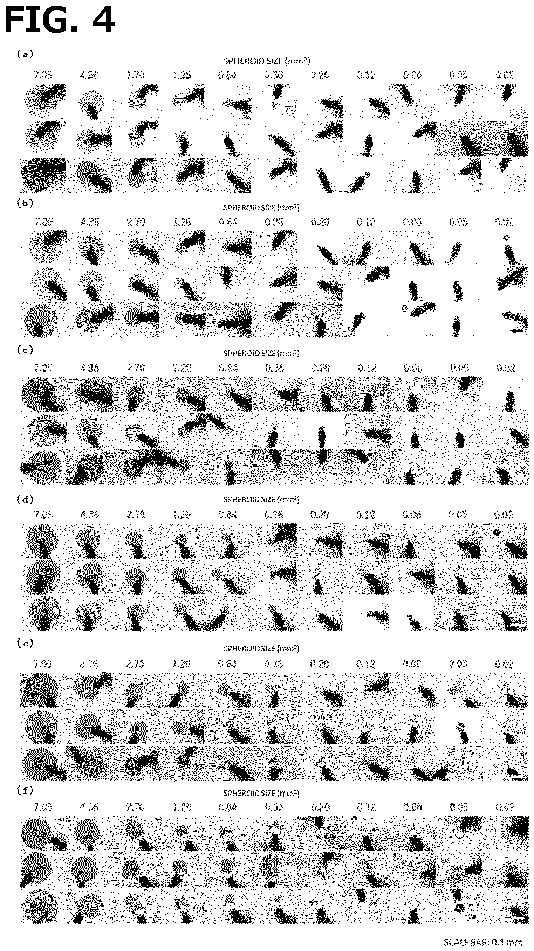

After 1 day from the start of culture, a state in each well was observed with an optical microscope, and it was evaluated whether or not the cells were held in LithoLoops Round. Fig. 4 shows the state in each well at this time {the area of the planned holding region of the frame member is (a) 1256 µm²; (b) 5024 µm²; (c) 31400 µm²; (d) 125600 µm²; (e) 282600 µm²; and (f) 502400 µm²}.

When the area of the planned holding region of the frame member was 1256 µm², all of the cells with any spheroid size were held in the frame member. Similarly, the cells were held in the frame member under the conditions of all of the cells with a spheroid size of 0.20 mm² or more when the area of the planned holding region was 5024 µm²; all of the cells with a spheroid size of 0.36 mm² or more when the area of the planned holding region was 31400 µm², 125600 µm², and 282600 µm²; and all of the cells with a spheroid size of 0.64 mm² or more when the area of the planned holding region was 502400 µm². Table 1 shows the presence or absence of cell holding under each condition.

**[Table 1]**

| Spheroid Size [mm2] | Area of Planned Holding Region of Frame Member [µm²] | | | | | |
|---|---|---|---|---|---|---|
| | 1 2 5 6 | 5 0 2 4 | 3 1 4 0 0 | 1 2 5 6 0 0 | 2 8 2 6 0 0 | 5 0 2 4 0 0 |
| 0.02 | ○ | × | × | × | × | × |
| 0.05 | ○ | × | × | × | × | × |
| 0.06 | ○ | × | × | × | × | × |
| 0.12 | ○ | × | × | × | × | × |
| 0.20 | ○ | ○ | × | × | × | × |
| 0.36 | ○ | ○ | ○ | ○ | ○ | × |
| 0.64 | ○ | ○ | ○ | ○ | ○ | ○ |
| 1.26 | ○ | ○ | ○ | ○ | ○ | ○ |
| 2.70 | ○ | ○ | ○ | ○ | ○ | ○ |
| 4.36 | ○ | ○ | ○ | ○ | ○ | ○ |
| 7.05 | ○ | ○ | ○ | ○ | ○ | ○ |

### <Evaluation of occupancy rate of planned holding region and holding period of cells>

Each LithoLoops Round, which was observed in the above evaluation of cell holding, was taken out, and left to stand in each medium of a 24-well plate (product number: MS-80120, Sumitomo Bakelite Co., Ltd.) to which 2 mL of the medium per well was added, the plate was left to stand in an incubator, and the culture was continued under the conditions of 37°C, and 5% CO₂.

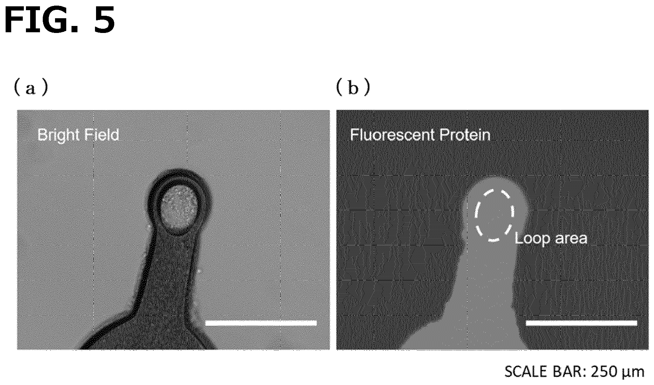

In each LithoLoops Round, a bright field image and a fluorescence image were obtained by using an optical microscope every predetermined period from the start of culture. As an example of the obtained image, Fig. 5 shows a bright field image (a) and a fluorescence image (b), when the area of the planned holding region of the frame member after 28 days from the start of culture was 5024 µm². Further, the "Loop area" shown in Fig. 5(b) is a planned holding region of the frame member. From this result, it was confirmed that the cell population was held in the frame member while maintaining the fluorescence activity. Further, at this time, all of the frame member was covered with the cell population. In other words, it was confirmed that the outer periphery was covered with the cell population in the entire cross section of the frame member perpendicular to a surface of the planned holding region.

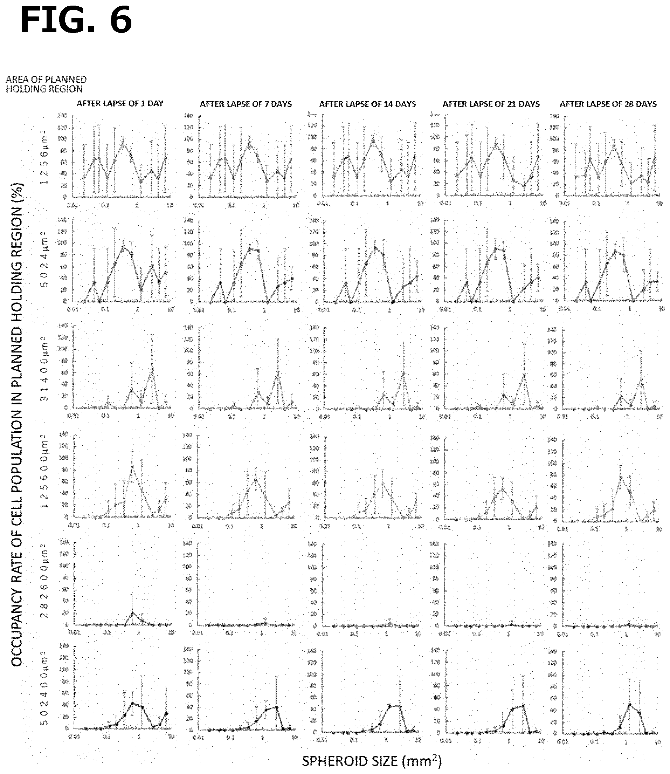

Subsequently, from these images, an occupancy rate of the cell population in the area of the planned holding region was calculated by using analysis software. Fig. 6 shows an occupancy rate of each cell population in the planned holding regions under each condition. In each graph, the horizontal axis represents a spheroid size, and the vertical axis represents an occupancy rate of the cell population in the planned holding region.

From the results shown in Fig. 6, under the conditions that the area of the planned holding region of the frame member was 1256 µm² and 5024 µm² and further the spheroid size was 0.20 mm², 0.36 mm², and 0.64 mm², the occupancy rate of the cells in the planned holding region until 28 days from the start of culture was 50% or more. In particular, under the conditions that the area of the planned holding region of the frame member was 1256 µm² and further the spheroid size was 0.36 mm², and under the conditions that the area of the planned holding region of the frame member was 5024 µm² and further the spheroid size was 0.36 mm² and 0.64 mm², the occupancy rate of the cells in the planned holding region until 28 days from the start of culture was 80% or more. Further, the area of the planned holding region of the frame member was 1256 µm², the spheroid size was 0.36 mm², and further until 14 days from the start of culture, and the area of the planned holding region of the frame member was 5024 µm², the spheroid size was 0.36 mm², and further until 21 days from the start of culture, the occupancy rate of the cells in the planned holding region was 90% or more. From the above, it was confirmed that the cell population was held in the planned holding region of the cell holding material over a long period of 14 days or more after the start of culture.

### «Holding of cultured primary cells by frame member made of polyimide»

### <Evaluation of relationship between the number of cells and spheroid size>

In other cell strains, in order to evaluate whether or not the cell population was held in the planned holding region of the cell holding material, the following tests were performed by using liver-derived cells. First, in order to evaluate the relationship between the number of seeded cells and a spheroid size, formation of a spheroid of liver-derived cells excised from a chicken fetus (E12) was induced by using a culture container to which a non (low)-cell adhesion treatment was performed. Specifically, in order to obtain the liver-derived cells, at first, a chicken fetus was taken out from a chicken egg on day 12 of incubation, and the liver was excised. Next, the excised liver was dispersed with a cell strainer having a mesh size of 100 µm (product number: 93100, SPL Life Science), and cells derived from the liver was obtained. A predetermined number of the cells were suspended and seeded in DMEM containing 10% FBS, and the culture was performed under the conditions of 37°C, and 5% CO₂.

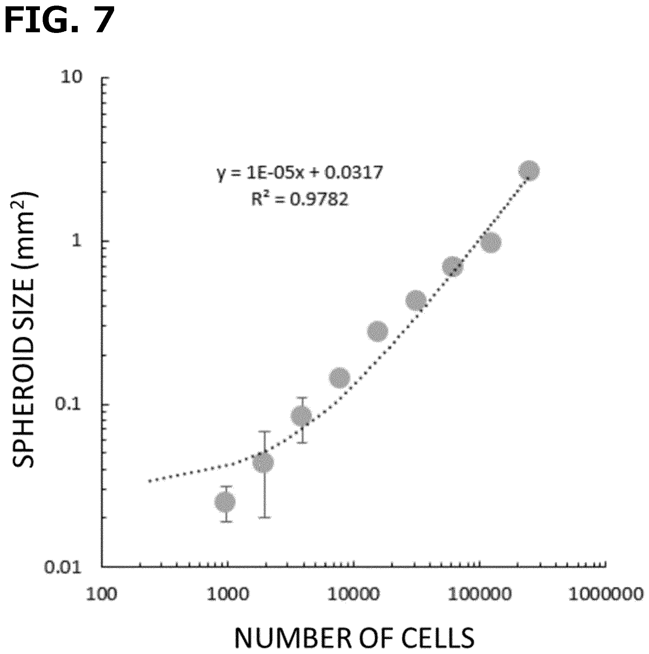

After 1 day from the start of culture, it was confirmed that a spheroid was formed in all of any number of cells. The spheroid size at this time was measured using analysis software from a photograph taken with an optical microscope. Results at this time of the number of seeded cells and the spheroid size in a logarithmic graph are shown in Fig. 7.

From the results shown in Fig. 7, when the number of seeded cells and the spheroid size were plotted on a logarithmic graph, it was found that there was a linear relationship between them even in the liver-derived cells of a chicken fetus (E12), similar to that of fluorescent protein-expressing HEK293T cells.

### <Evaluation of cell holding>

Liver-derived cells of a chicken fetus (E12) were suspended in DMEM containing 10% FBS, and seeded in 2 wells of a culture container to which a non (low)-cell adhesion treatment was performed so that a spheroid size is 0.20 mm². At the same time, as the frame member, LithoLoops Rounds having areas of the planned holding region of 1256 µm² and 5024 µm² were immersed in the wells in each of which the cells were seeded, respectively. This plate was left to stand in an incubator, the culture was started under the conditions of 37°C, and 5% CO₂.

After 1 day from the start of culture, each LithoLoops Round was taken out, and left to stand in each medium of a 24-well plate to which 2 mL of the medium per well was added. This plate was left to stand in an incubator, the culture was continued under the conditions of 37°C, and 5% CO₂.

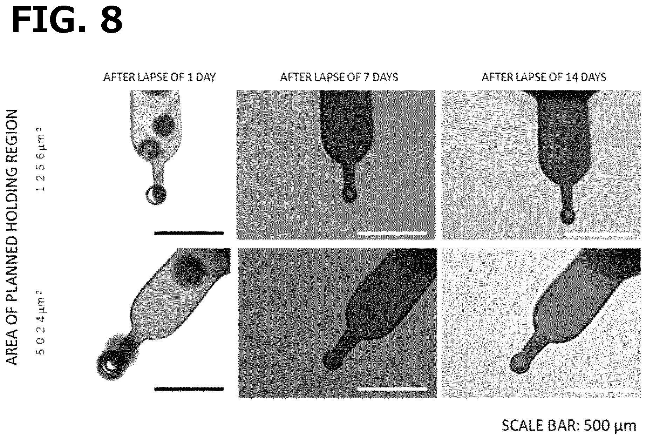

A state in each well was observed with an optical microscope every predetermined period from the start of culture to evaluate whether or not the cells were held in the frame member. Fig. 8 shows the state in each well at this time.

From the results shown in Fig. 8, when the spheroid size of liver-derived cells of a chicken fetus (E12) was 0.20 mm² and further the area of the planned holding region of the frame member was 1256 µm² and 5024 µm², the cells were held in the frame member. Further, it was confirmed that the cell population was held in the planned holding region of the frame member until 14 days from the start of culture under any conditions.

### <Evaluation of cells derived from various organs>

Further, for other cell strains, it was evaluated whether or not the cell population was held in the planned holding region of the cell holding material. Specifically, at first, cells derived from a liver, a stomach, a lung, a heart, and a brain were obtained in a similar manner to the above-described liver-derived cells. The number of cells, with which a spheroid size with the highest cell holding rate after 28 days from the start of culture was obtained in Fig. 6, was set as the number of the cells to be seeded derived from each organ. Table 2 shows the number of cells to be set for the cells derived from each organ. Herein, as shown in Table 2, the number of cells to be set of the brain-derived cells was different from that of the cells derived from other organs. From these results, it was indicated that the area of the planned holding region that can be occupied by brain-derived cells may be different from the areas of the planned holding regions that can be occupied by other cells.

**[Table 2]**

| Area of Planned Holding Region of Frame Member [µm²] | The Number of Cells to be Set | |
|---|---|---|
| | Liver, Stomach, Lung, and Heart | Brain |
| 1 2 5 6 | 3.29×10⁴ | 5.59×10⁴ |
| 5 0 2 4 | 3.29×10⁴ | 5.59×10⁴ |
| 3 1 4 0 0 | 26.82×10⁴ | 49.18×10⁴ |
| 1 2 5 6 0 0 | 6.10×10⁴ | 10.80×10⁴ |
| 2 8 2 6 0 0 | 12.39×10⁴ | 22.44×10⁴ |
| 5 0 2 4 0 0 | 12.39×10⁴ | 22.44×10⁴ |

The cells derived from each organ with the number of cells set above were suspended in DMEM containing 10% FBS, and seeded in 2 wells of a culture container to which a non (low)-cell adhesion treatment was performed. At the same time, as the frame member, LithoLoops Rounds having predetermined areas of the planned holding region were immersed in the wells in each of which the cells were seeded, respectively. This plate was left to stand in an incubator, the culture was started under the conditions of 37°C, and 5% CO₂.

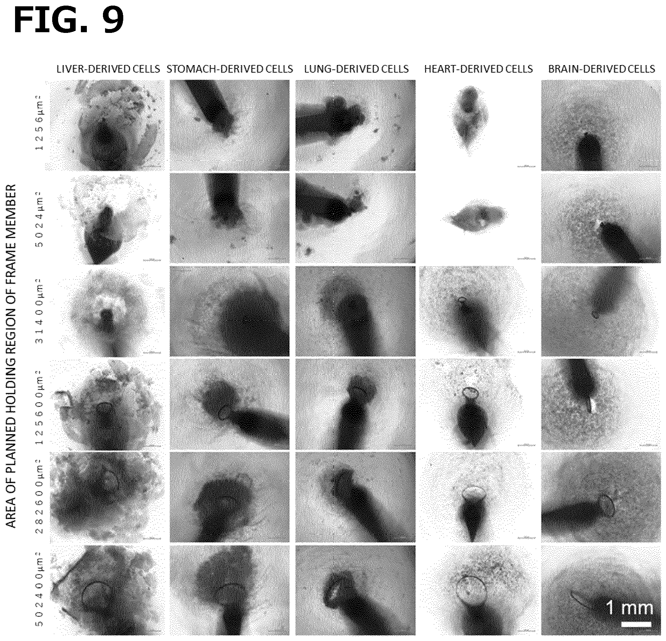

After 1 day from the start of culture, a state in each well is shown in Fig. 9. All of the cells derived from any organ, which have any area of the planned holding region of the frame member and further any spheroid size, were also held in the frame member. Subsequently, each LithoLoops Round was taken out, and left to stand in each medium of a 24-well plate to which 2 mL of the medium per well was added. This plate was left to stand in an incubator, the culture was continued under the conditions of 37°C, and 5% CO₂.

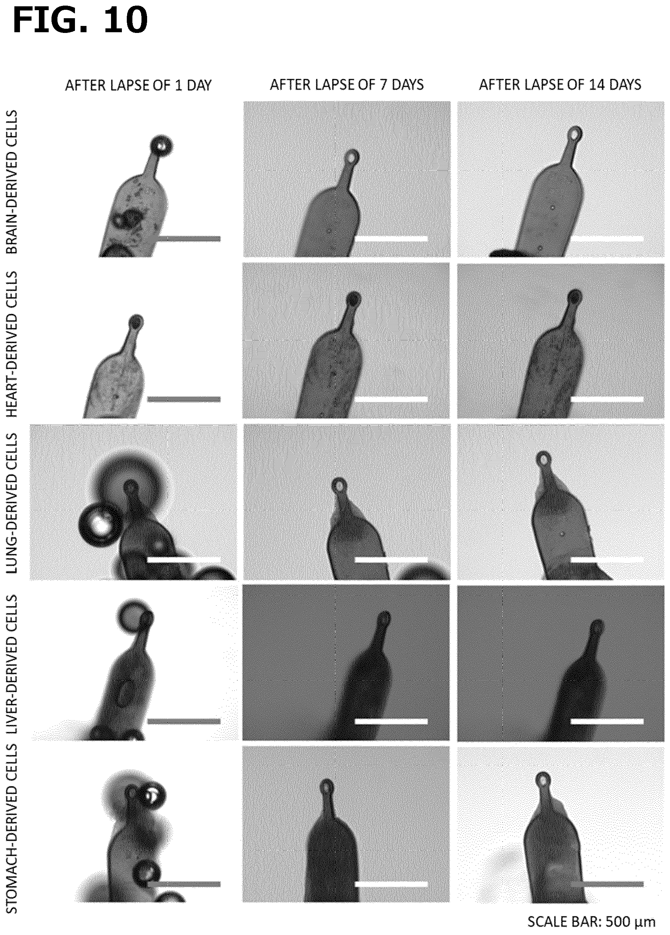

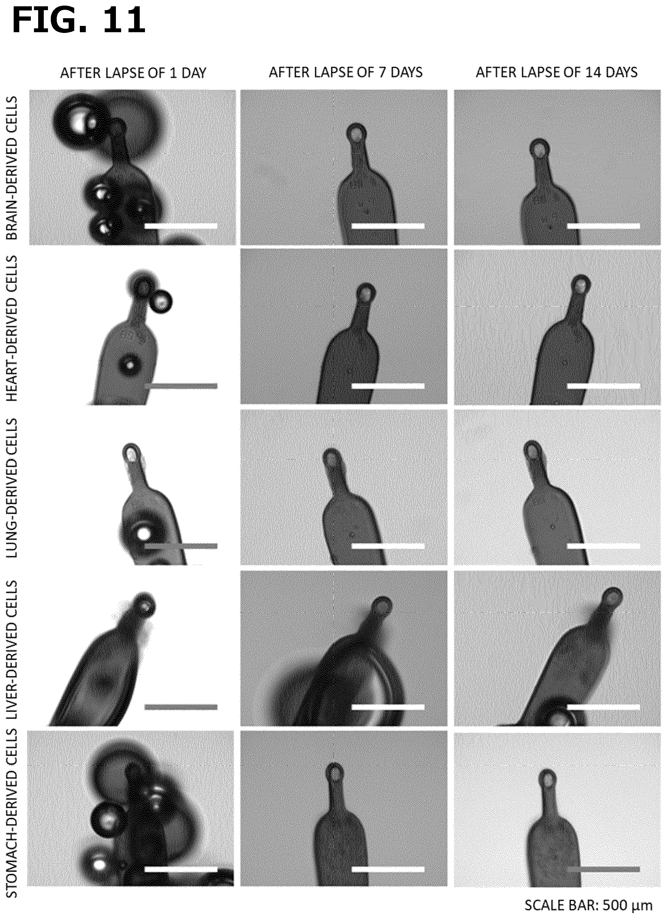

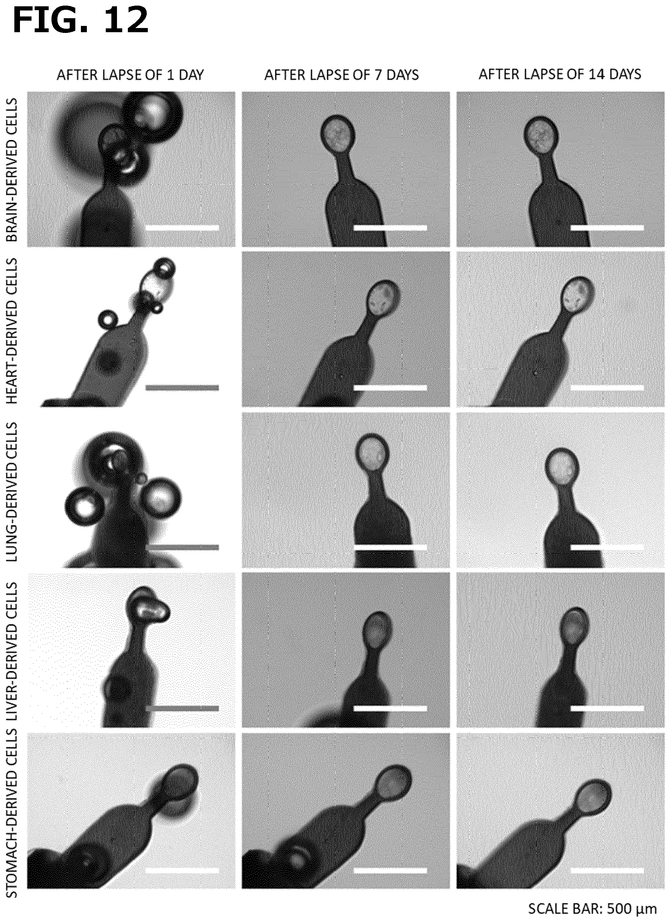

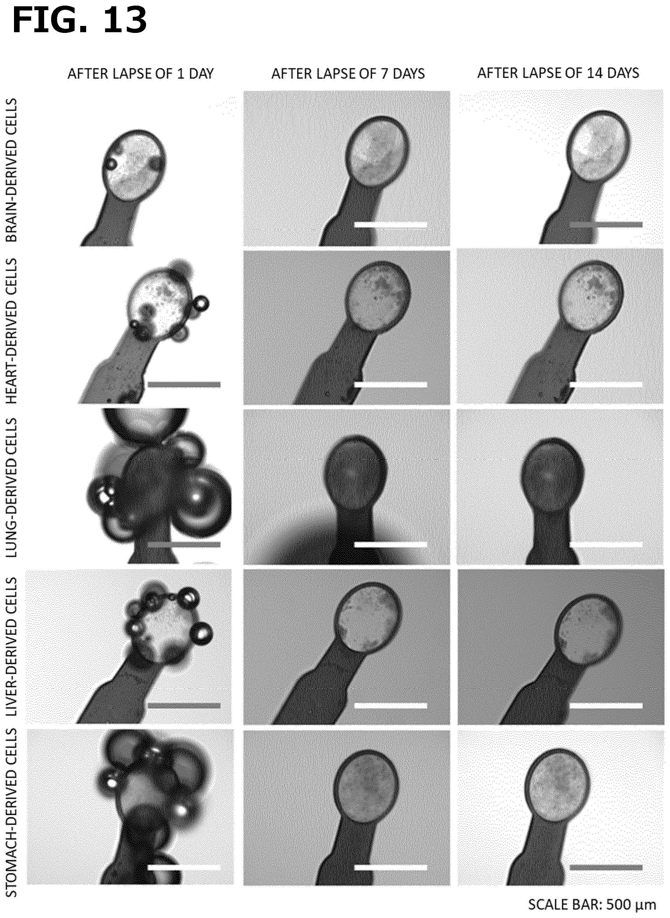

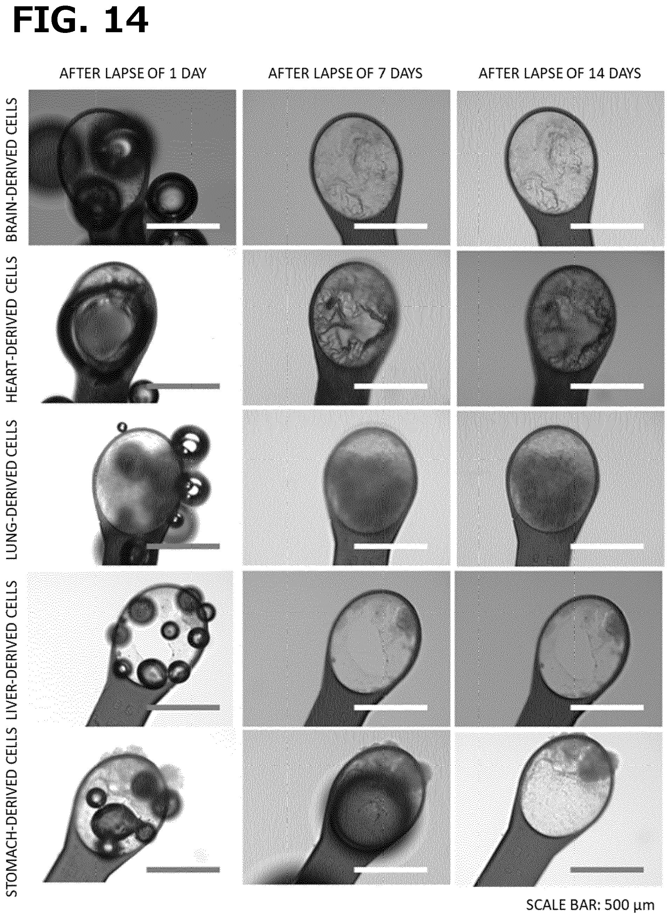

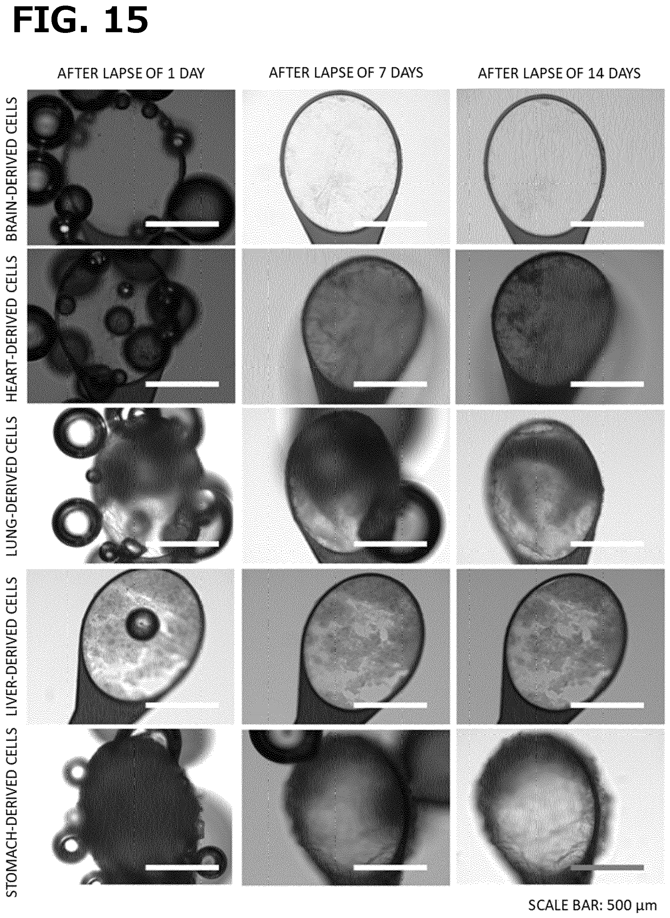

For each LithoLoops Round, a bright field image was obtained by using an optical microscope every predetermined period from the start of culture. Bright field images after 1, 7, and 14 days from the start of culture when the area of the planned holding region of the frame member was 1256 µm² (Fig. 10), 5024 µm² (Fig. 11), 31400 µm² (Fig. 12), 125600 µm² (Fig. 13), 282600 µm² (Fig. 14), and 502400 µm² (Fig. 15) are shown.

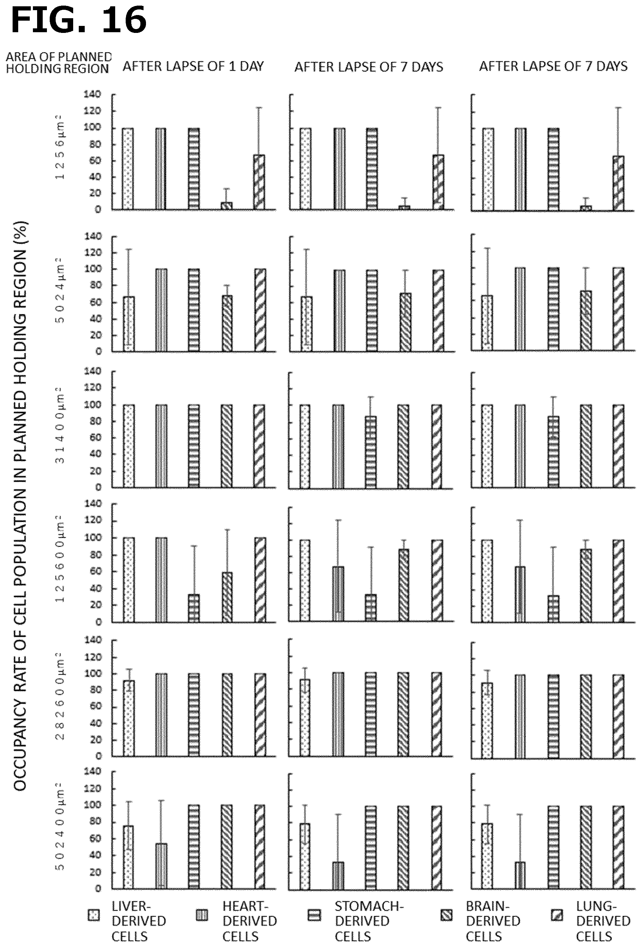

Subsequently, from these images, an occupancy rate of the cell population in the area of the planned holding region was calculated by using analysis software. At this time, in each image, the calculation was performed by excluding a portion where bubbles overlapped in the planned holding region. Fig. 16 shows an occupancy rate of each cell population in the planned holding region under each condition. The vertical axis in each graph represents the occupancy rate of the cell population in the planned holding region. Further, Table 3 shows the occupancy rate in the planned holding region after 14 days from the start of culture in each of the cells. In addition, for the HEK293T cells, the occupancy rate in the number of cells, with which a spheroid size with the highest cell holding rate after 28 days from the start of culture was obtained in Fig. 6, is shown.

**[Table 3]**

| Area of Planned Holding Region of Frame Member [µm²] | 1 2 5 6 | 5 0 2 4 | 3 1 4 0 0 | 1 2 5 6 0 0 | 2 8 2 6 0 0 | 5 0 2 4 0 0 |
|---|---|---|---|---|---|---|
| HEK293TCells | 94.3% | 92.9% | 61.8% | 59.2% | 4.3% | 45.4% |
| Liver-Derived Cells | 100% | 66.7% | 100% | 100% | 92.9% | 75.4% |
| Heart-Derived Cells | 100% | 100% | 100% | 100% | 100% | 54.5% |
| Stomach-Derived Cells | 100% | 100% | 100% | 33.3% | 100% | 100% |
| Brain-Derived Cells | 9.2% | 68.1% | 100% | 58.5% | 100% | 100% |
| Lung-Derived Cells | 66.7% | 100% | 100% | 100% | 100% | 100% |

From the results shown in Table 3, even when the area of the planned holding region of the frame member was 282600 µm² and 502400 µm² where the occupancy rate in the planned holding region was less than 50% in the HEK293T cells, the occupancy rate in the planned holding region after 14 days from the start of culture was 50% or more even in the cells derived from any organ. In particular, the cell population occupied all the planned holding region after 14 days from the start of culture under the conditions of heart-, stomach-, brain-, and lung-derived cells when the area of the planned holding region of the frame member was 282600 µm² and under the conditions of stomach-, brain-, and lung-derived cells when the area was 502400 µm².

### «Holding of HEK293T cells by frame member made of sponge»

In addition to the frame member made of polyimide, in order to evaluate whether or not the cell population was held in the planned holding region of the frame member, the following tests were performed by using a spongy sponge (frame member made of sponge, cross-sectional diameter of frame structure body: about 30 to 60 µm). Fluorescent protein-expressing HEK293T cells were suspended in DMEM containing 10% FBS, and seeded in a culture container to which a non (low)-cell adhesion treatment was performed so that a spheroid size is 0.36 mm². At the same time, as the frame member, a spongy sponge (ROSY ROSA) was immersed in each well in which the cells were seeded. This plate was left to stand in an incubator, the culture was started under the conditions of 37°C, and 5% CO₂.

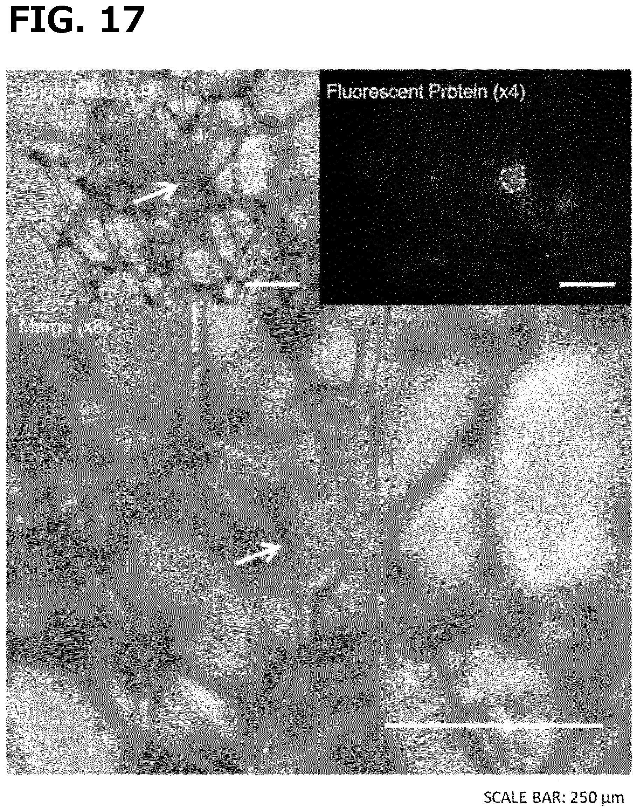

A state in each well was observed with an optical microscope after 1 day from the start of culture, and it was confirmed that the cells were held in the frame member. Subsequently, each of the cell holding materials observed was taken out, and left to stand in each medium of a 24-well plate to which 2 mL of the medium per well was added. For the state of the spongy sponge at this time, a bright field image and a fluorescence image were obtained by using an optical microscope. Fig. 17 shows a bright field image (upper left), a fluorescence image (upper right), and a merged image thereof (bottom) at this time.

From the results shown in Fig. 17, it was confirmed that the cell population was held in part of the frame structure body of the spongy sponge. Further, for this frame structure body, the area of the planned holding region formed by the frame structure body and the occupancy rate of the cell population in the area of the planned holding region were calculated by using analysis software. As a result, the area of the planned holding region formed by the frame structure body was 4717 µm², and the occupancy rate of a cell population in the area of the planned holding region was 100%. From the above, it was indicated that the cells were held also by the frame member made of sponge.

### Industrial Applicability

The present invention can be used in production of a cell holding material that can be used as a cell sensor utilizing physiological activity of cells, a tool for regenerative medicine or pharmacokinetic evaluation, or the like.

### Reference Signs List

- 10: Cell holding material
- 20: Frame member
- 22: Planned holding region
- 24: Bar member
- 30: Cell population

## Claims

1. A cell holding material, comprising:
a frame member for surrounding all or at least part of a planned holding region capable of holding a cell population obtained by aggregating a plurality of cells; and
the cell population in a form of membrane occupying at least part of the planned holding region and being held by the frame member,
wherein at least part of a margin of the cell population is brought into contact with the frame member, and further a portion occupying the planned holding region in the cell population is hung without physical support.

2. The cell holding material according to claim 1, wherein an outer periphery of the frame member in a cross section perpendicular to a surface of the planned holding region is at least partially covered with the cell population.

3. The cell holding material according to claim 1 or 2, wherein the cell population occupies 50% or more of an area of the planned holding region.

4. The cell holding material according to any one of claims 1 to 3, wherein the planned holding region is a substantially circular shape or a substantially elliptical shape.

5. The cell holding material according to any one of claims 1 to 4, wherein the frame member surrounds all of the planned holding region.

6. The cell holding material according to any one of claims 1 to 5, wherein cells of the cell population are cultured primary cells.

7. The cell holding material according to any one of claims 1 to 6, wherein cells of the cell population are cells derived from an organ selected from a liver, a stomach, a lung, a heart, or a brain.

8. The cell holding material according to claim 6 or 7, wherein an area of the planned holding region is 1000 to 600000 µm².

9. The cell holding material according to any one of claims 1 to 8, wherein an area of the planned holding region is 1000 to 6000 µm².

10. The cell holding material according to any one of claims 1 to 9, wherein a spheroid formed of the cell population has a spheroid size of 0.2 to 1.0 mm².

11. A sensor, comprising the cell holding material according to any one of claims 1 to 10.

12. A frame member for holding part of a cell population obtained by aggregating a plurality of cells while hanging the part without physical support,
wherein the frame member surrounds all or at least part of a planned holding region capable of holding the cell population.

13. The frame member according to claim 12, wherein an area of the planned holding region is 1000 to 6000 µm².

14. A cell holding method for holding part of a cell population obtained by aggregating a plurality of cells while hanging the part without physical support, comprising
immersing a frame member for surrounding all or at least part of a planned holding region of the cell population in a medium containing the cell population or a spheroid formed of the cell population.

15. The cell holding method according to claim 14, wherein an area of the planned holding region is 1000 to 6000 µm².

16. The cell holding method according to claim 14 or 15, wherein a spheroid formed of the cell population has a spheroid size of 0.2 to 1.0 mm².
